# EUROPEAN PATENT APPLICATION

(11) **EP 4 652 983 A1**
(43) Date of publication of application: **26.11.2025**
(21) Application number: 24744543.0
(22) Date of filing: 10.01.2024
(51) Int. Cl.: A61K 8/22, A61K 8/34, A61K 8/41, A61K 8/44, A61K 8/81, A61K 8/86, A61Q 5/08, A61Q 5/10

(54) **COSMETIC PRODUCT FOR HAIR BLEACHING OR HAIR DYEING**

(30) Priority: 19.01.2023 JP 2023006595
(71) Applicant: Kao Corporation, Chuo-ku, Tokyo 103-8210 (JP)
(72) Inventor: UEHARA, Takuya, Tokyo 131-8501 (JP); SAKAI, Yuta, Tokyo 131-8501 (JP); KANDA, Takashi, Tokyo 131-8501 (JP)
(74) Representative: Hoffmann Eitle
(86) International application number: PCT/JP2024/000241
(87) International publication number: WO 2024/154620

(57) **Abstract**

The present invention provides a cosmetic product for bleaching or dyeing hair as a foam that allows agents to sufficiently permeate to the deep (adjacent to the roots) of the head hair and gives a finish without color unevenness between the surface and the deep of the head hair.

That is, the present invention is a cosmetic product for bleaching or dyeing hair, comprising a first part containing an alkali agent, a second part containing hydrogen peroxide, and a container for dispensing a liquid mixture including the first part and the second part as a foam, wherein
at least one of all agents, including the first part and the second part, before mixing is a solubilizing solution,
the liquid mixture contains components (A) to (D) below, and
assuming the component (A) contained in the solubilizing solution before mixing as component (A)s, the mass ratio of the component (A)s to the sum of the component (A) and the component (B), (A)s/[(A) + (B)], is 0.079 or less,
(A) an aliphatic alcohol having 10 or more and 14 or less carbon atoms;
(B) an aliphatic alcohol having 19 or more and 24 or less carbon atoms;
(C) an ionic surfactant; and
(D) a polymer including a diallyl quaternary ammonium salt as a constituting unit.

## Description

### Field of the Invention

The present invention relates to a cosmetic product for bleaching or dyeing hair.

### Background of the Invention

In recent years, in hair bleach or hair dye, it has been proposed to simplify a hair bleaching or dyeing process by dispensing an agent as a foam. For example, a one-part or two-part aerosol or non-aerosol type product is known. However, to apply a conventional foam formulation to hair to which sebum, out-bath type hair treatment, hair conditioner, or the like adhere, the foam may disappear easily when the dispensed foam is applied to the hair or held on the head hair, and also dripping may easily occur. In such a case, there is difficulty in dyeing the hair evenly and neatly.

In order to solve this problem, it is known an agent that has good foaming and foam retention and allows bleaching or dyeing without color unevenness, even if applied to the head hair with a hair cosmetic, by employing a specific anionic surfactant and a specific cationic polymer in a liquid mixture of the first part and the second part in a non-aerosol type hair bleaching or hair dyeing cosmetic product (Patent Literature 1).

Patent Literature 1: JP-A-2015-107962

### Summary of the Invention

The present invention provides a cosmetic product for bleaching or dyeing hair, comprising a first part containing an alkali agent, a second part containing hydrogen peroxide, and a container for dispensing a liquid mixture including the first part and the second part as a foam, wherein
at least one of all agents, including the first part and the second part, before mixing is a solubilizing solution,
the liquid mixture contains components (A) to (D) below, and
assuming the component (A) contained in the solubilizing solution before mixing as component (A)s, a mass ratio of the component (A)s to the sum of the component (A) and the component (B), (A)s/[(A) + (B)], is 0.079 or less,
   (A) an aliphatic alcohol having 10 or more and 14 or less carbon atoms,
   (B) an aliphatic alcohol having 19 or more and 24 or less carbon atoms,
   (C) an ionic surfactant, and
   (D) a polymer including a diallyl quaternary ammonium salt as a constituting unit.

Furthermore, the present invention provides a hair bleaching or hair dyeing method comprising using the above cosmetic product for bleaching or dyeing hair to dispense a liquid mixture containing the first part and the second part as a foam from the container, and applying the foam to hair with a hand.

Even in the technique described in Patent Literature 1, there is difficulty in permeation of an agent to the deep (adjacent to the roots) of the head hair in accordance with the foam-applying method by a user. That is, a user who is not very skilled in bleaching or coloring, for example, a general consumer who has just started using hair color for home use tends to "smooth down" the dispensed foam to the hair. In such a case, the foam does not sufficiently permeate to the deep of the head hair, and an undyed part or uneven dyeing is likely to occur. In addition, for consumers who are not satisfied with the finish and desire a better finish, excellent permeability to the deep of the head hair is a desirable characteristic as a hair bleach or hair dye as a foam.

Accordingly, the present invention relates to a cosmetic product for bleaching or dyeing hair as a foam in which the agent sufficiently permeates to the deep (adjacent to the roots) of the head hair to give a finish without color unevenness between the surface and the deep of the head hair, while taking advantage of the technical features described in Patent Literature 1.

The present inventors found that in a multiple-agent type cosmetic product for bleaching or dyeing hair by dispensing a liquid mixture containing a first part containing an alkali agent and a second part containing hydrogen peroxide as a foam from a container, the viscosity of the foam during the period from the dispense to the application is maintained low, the permeability to the deep (adjacent to the roots) of the head hair and the transferability of the liquid mixture components into the inside of hair fibers are significantly improved, and further the stability of the foam against oily components such as sebum are also improved by constituting all agents before mixing from at least one solubilizing solution and at least one emulsion, using two groups of aliphatic alcohols having specific chain lengths, an ionic surfactant, and a specific cationic polymer in combination, and suppressing the relative amount of a short-chain aliphatic alcohol in the solubilizing solution to the total amount of the aliphatic alcohols of the two groups in the liquid mixture. As a result, the present invention exhibits high bleachability or dyeability without color unevenness between the deep and the surface of the head hair.

The cosmetic product for bleaching or dyeing hair of the present invention has significantly improved permeability of dispensed foam to the deep (near the scalp) of the hair of head and transferability of the liquid mixture components into the inside of hair fibers and further has excellent stability of foam against oily components such as sebum and, as a result, exhibits high bleachability or dyeability without color unevenness between the deep and the surface of the head hair.

### Detailed Description of the Invention

The cosmetic product for bleaching or dyeing hair of the present invention can be used as a two-part cosmetic product to be used by mixing a first part and a second part or can be used as a three-part or four-part cosmetic product to be used by further mixing an agent containing a granulated material of a persulfate or the like or an agent containing a conditioning component as a third part or a fourth part, in addition to a first part and a second part. In the present invention, the liquid mixture means a liquid mixture of a first part and a second part in a two-part cosmetic product, a liquid mixture of a first part, a second part, and a third part in a three-part cosmetic product, and a liquid mixture of a first part, a second part, a third part, and a fourth part in a four-part cosmetic product.

Among all the agents, before mixing, constituting a product for bleaching or dyeing of the present invention including the first part and the second part, at least one agent is a solubilizing solution, and the remaining agents may be any of a solubilizing solution or an emulsion, and preferably include at least one emulsion.

The solubilizing solution means a thermodynamically stable isotropic solution prepared by adding an amphiphilic component to a material that is normally insoluble or slightly soluble in a certain solvent as described in Reference Literature 1 (Moroi, Y., "Solubilization", Micelles, 1992, chapter 9, pp. 167-181 (URL: https://doi.org/10.1007/978-1-4899-0700-4_9)).

### [Alkali agent]

The first part contains an alkali agent. Examples of the alkali agent include ammonia and its salt, such as strong ammonia water and ammonium chloride; an alkanolamine and its salt, such as monoethanolamine, isopropanolamine, 2-amino-2-methylpropanol, and 2-aminobutanol; an alkanediamine and its salt, such as 1,3-propanediamine; and a carbonate such as guanidine carbonate, sodium carbonate, potassium carbonate, sodium hydrogen carbonate, and potassium hydrogen carbonate. These alkali agents may be used in combination of two or more. The content of the alkali agent in the liquid mixture is preferably 1.0 mass% or more, more preferably 1.5 mass% or more, and further more preferably 2.0 mass% or more from the standpoint of a sufficient bleaching or dyeing effect and is preferably 20 mass% or less, more preferably 15 mass% or less, further more preferably 10 mass% or less, and further more preferably 5 mass% or less from the standpoint of reductions in fiber damage and scalp irritation.

### [Hydrogen peroxide]

The second part contains hydrogen peroxide. The content of hydrogen peroxide in the second part is preferably 0.5 mass% or more, more preferably 1 mass% or more, and further more preferably 3 mass% or more and is preferably 12 mass% or less, more preferably 9 mass% or less, and further more preferably 6 mass% or less. The content of hydrogen peroxide in the liquid mixture is preferably 0.5 mass% or more, more preferably 1 mass% or more, and further more preferably 2 mass% or more and is preferably 6 mass% or less and more preferably 5 mass% or less. The pH of the second part is preferably 2 or more and more preferably 2.5 or more and is preferably 6 or less and more preferably 4 or less, from the viewpoint of suppressing decomposition of hydrogen peroxide.

### [Component (A): aliphatic alcohol having 10 or more and 14 or less carbon atoms]

The cosmetic product for bleaching or dyeing hair of the present invention contains an aliphatic alcohol having 10 or more and 14 or less carbon atoms as a component (A) in the liquid mixture. The component (A) is preferably a saturated aliphatic alcohol and is preferably a linear aliphatic alcohol. The number of carbon atoms of the component (A) is preferably from 12 to 14 and more preferably 14.

Examples of the component (A) include decanol, dodecanol, and myristyl alcohol, and myristyl alcohol is particularly preferable.

The aliphatic alcohols as the component (A) can be used alone or in combination of two or more. The content of the component (A) in the liquid mixture is preferably 0.1 mass% or more, more preferably 0.15 mass% or more, and further more preferably 0.2 mass% or more and is preferably 2 mass% or less, more preferably 1 mass% or less, and further more preferably 0.5 mass% or less, from the viewpoint of enhancing the permeability to the deep of the head hair, the transferability into the inside of hair fibers, and the serum resistance to realize high bleachability or dyeability without color unevenness.

A part of the component (A) can be contained in the solubilizing solution before mixing. In the present invention, in the component (A), the component (A) contained in the solubilizing solution before mixing is referred to as "component (A)s".

### [Component (B): aliphatic alcohol having 19 or more and 24 or less carbon atoms]

The cosmetic product for bleaching or dyeing hair of the present invention contains an aliphatic alcohol having 19 or more and 24 or less carbon atoms as a component (B) in the liquid mixture. The component (B) is preferably a saturated aliphatic alcohol and is preferably a linear aliphatic alcohol. The number of carbon atoms of the component (B) is preferably 20 to 24 and more preferably 22.

Examples of the component (B) include arachyl alcohol, behenyl alcohol, and carnaubyl alcohol, and behenyl alcohol is particularly preferable.

The aliphatic alcohols as the component (B) can be used alone or in combination of two or more. The content of the component (B) in the liquid mixture is preferably 0.05 mass% or more, more preferably 0.1 mass% or more, and further more preferably 0.2 mass% or more and is preferably 2 mass% or less, more preferably 1.5 mass% or less, and further more preferably 1 mass% or less, from the viewpoint of enhancing the permeability to the deep of the head hair, the transferability into the inside of hair fibers, and the serum resistance to realize high bleachability or dyeability without color unevenness.

The cosmetic product for bleaching or dyeing hair of the present invention preferably does not contain the component (A), the component (B), or an aliphatic alcohol other than these components in the solubilizing solution from the viewpoint of enhancing the permeability to the deep of the head hair, the transferability into the inside of hair fibers, and the serum resistance to realize high bleachability or dyeability without color unevenness, and even if these aliphatic alcohols are included, the total content thereof is preferably as less as possible and is preferably 0.3 mass% or less, more preferably 0.2 mass% or less, further more preferably 0.1 mass% or less, further more preferably 0.05 mass% or less, further more preferably 0.01 mass% or less, further more preferably 0.005 mass% or less, and further more preferably 0.001 mass% or less.

In the cosmetic product for bleaching or dyeing hair of the present invention, assuming the component (A) contained in the solubilizing solution before mixing as component (A)s, the component (A)s has a specific quantitative relation with the total of the component (A) and the component (B). That is, the mass ratio showing the ratio of the amount (mass%) of the component (A)s based on 100 mass% of the total amount of the component (A) and the component (B) contained in the liquid mixture obtained in accordance with the appropriately determined mix ratio of the agents including the first part and the second part, (A)s/[(A) + (B)], shows a specific value.

Specifically, in the cosmetic product for bleaching or dyeing hair of the present invention, assuming the component (A) contained in the solubilizing solution before mixing as component (A)s, from the viewpoint of enhancing the permeability to the deep of the head hair, the transferability into the inside of hair fibers, and the serum resistance to realize high bleachability or dyeability without color unevenness, the mass ratio of the component (A)s to the sum of the component (A) and the component (B), (A)s/[(A) + (B)], is 0.079 or less, preferably 0.07 or less, more preferably 0.06 or less, further more preferably 0.05 or less, further more preferably 0.04 or less, further more preferably 0.03 or less, further more preferably 0.02 or less, further more preferably 0.01 or less, further more preferably 0.005 or less, further more preferably 0.001 or less, further more preferably 0.0005 or less, and further more preferably 0.0001 or less. Furthermore, the solubilizing solution preferably does not contain the components (A), that is, the amount of the component (A)s in 100 mass% of the solubilizing solution is preferably 0 mass%.

When the cosmetic product for bleaching or dyeing hair of the present invention includes a plurality of solubilizing solutions, the amount of the component (A)s contained in the solubilizing solution is the total amount of the component (A) contained in all solubilizing solutions.

### [Component (C): ionic surfactant]

The cosmetic product for bleaching or dyeing hair of the present invention contains an ionic surfactant as a component (C) in the liquid mixture. Examples of the ionic surfactant include a cationic surfactant, an anionic surfactant, and an amphoteric surfactant. In particular, from the viewpoint of the foaming and foam retention of the dispensed liquid mixture and the solubility of the dye contained in the cosmetic product for bleaching or dyeing hair, a cationic surfactant and an anionic surfactant are preferable.

### <Cationic surfactant>

The cationic surfactant is preferably a long-chain monoalkylammonium salt of the following formula (1): 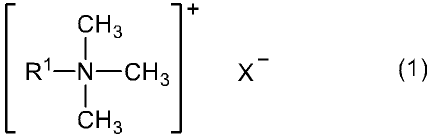 wherein, R¹ is a linear alkyl group having 12 to 24 carbon atoms, and X⁻ is an anion (such as a chloride ion, a bromide ion, an iodide ion, a sulfate anion, a sulfonate anion, a methyl sulfate anion, a phosphate anion, and a nitrate anion).

R¹ in the formula (1) preferably has 12 to 18 carbon atoms from the viewpoint of enhancing the permeability to the deep of the head hair, the transferability into the inside of hair fibers, and the serum resistance to realize high dyeability without color unevenness.

Examples of the more preferable cationic surfactant include a combination of a long-chain monoalkylammonium salt of the following formula (2) and a long-chain monoalkylammonium salt of the following formula (3): 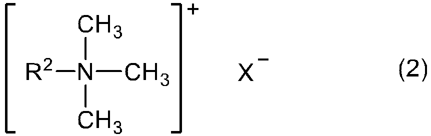 wherein, R² is a linear alkyl group having 12 to 16 carbon atoms, and X⁻ means the same as above, 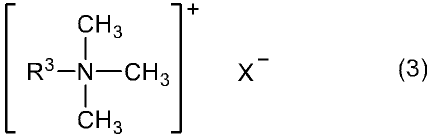 wherein, R³ is a linear alkyl group having 18 to 24 carbon atoms, and X⁻ means the same as above.

R² in the formula (2) preferably has 14 to 16, more preferably 16, carbon atoms from the viewpoint of enhancing the permeability to the deep of the head hair, the transferability into the inside of hair fibers, and the serum resistance to realize high dyeability without color unevenness. R³ in the formula (2) preferably has 18 to 20, more preferably 18, carbon atoms, from the same viewpoint.

Specific examples of the preferable cationic surfactant include cetyltrimethylammonium chloride and stearyltrimethylammonium chloride.

### <Anionic surfactant>

Examples of the anionic surfactant include a sulfate anionic surfactant, such as an alkyl sulfate and an alkyl ether sulfate; a carboxylic acid anionic surfactant, such as an N-acylamino acid salt, an N-acyl-N-alkylamino acid salt, an amide-type N-acylamino acid salt, an ether carboxylate, a fatty acid salt, an alkyl succinate, and an alkenyl succinate; a sulfonic acid anionic surfactant, such as sulfosuccinate type, isethionate type, taurine salt type, alkylbenzenesulfonate type, α-olefinsulfonate type, and alkanesulfonate type anionic surfactants; and a phosphate anionic surfactant, such as an alkyl phosphate and an alkyl ether phosphate. Among these surfactants, carboxylic acid-based and sulfate-based anionic surfactants are preferable. In the carboxylic acid anionic surfactants, an N-acylamino acid salt and an ether carboxylate are preferable. In particular, preferred are an N-acylglutamate of which the acyl group has carbon atoms of 10 to 18, preferably 10 to 16, and further more preferably 10 to 14 and a polyoxyethylene alkyl ether sulfate of which the alkyl group has carbon atoms of 10 to 18, preferably 10 to 16, and further more preferably 10 to 14 and the oxyethylene group has an average number of moles added of 4 or less, preferably 3 or less, and further more preferably 4 or less.

Specific examples of the preferable anionic surfactant include sodium cocoyl glutamate and sodium polyoxyethylene lauryl ether sulfate.

### <Amphoteric surfactant>

Examples of the amphoteric surfactant include carbobetaine, amidobetaine, sulfobetaine, hydroxysulfobetaine, amidosulfobetaine, phosphobetaine, and imidazolinium surfactants having an alkyl group, alkenyl group, or acyl group having 8 to 24 carbon atoms. In particular, a carbobetaine surfactant and a sulfobetaine surfactant are preferable. Examples of the preferable amphoteric surfactant include lauramidopropyl betaine, coconut oil fatty acid amidopropyl betaine, lauryldimethylaminoacetic acid betaine, and laurylhydroxysulfobetaine.

The ionic surfactants as the component (C) can be used alone or in combination of two or more. The total content of the component (C) in the liquid mixture is preferably 0.5 mass% or more, more preferably 1 mass% or more, further more preferably 1.5 mass% or more, and further more preferably 2 mass% or more and is preferably 7 mass% or less, more preferably 5 mass% or less, further more preferably 4 mass% or less, and further more preferably 3 mass% or less, from the viewpoint of enhancing the permeability to the deep of the head hair, the transferability into the inside of hair fibers, and the serum resistance to realize high bleachability or dyeability without color unevenness.

### [Component (D): polymer including diallyl quaternary ammonium salt as constituting unit]

The cosmetic product for bleaching or dyeing hair of the present invention contains a polymer including a diallyl quaternary ammonium salt as a constituting unit as a component (D) in the liquid mixture. As the polymer including a diallyl quaternary ammonium salt as a constituting unit in the present invention, a polymer that further includes a cationic group other than the diallyl quaternary ammonium salt or another anionic group is also included. In also such a polymer, only the charge density of cationic groups is considered.

The charge density of the component (D) is preferably 4.0 meq/g or more, more preferably 4.3 meq/g or more, further more preferably 4.6 meq/g or more, further more preferably 4.9 meq/g or more, further more preferably 5.2 meq/g or more, and further more preferably 5.5 meq/g or more and is preferably 8.0 meq/g or less, more preferably 7.0 meq/g or less, and further more preferably 6.5 meq/g or less, from the viewpoint of enhancing the permeability to the deep of the head hair, the transferability into the inside of hair fibers, and the serum resistance to realize high dyeability without color unevenness. Here, the charge density in the present invention refers to (the number of moles of cationic groups per gram of polymer) × 1000 (meq/g).

The polymer of the component (D) is a polymer having a skeleton of the following formula (4) or (5): 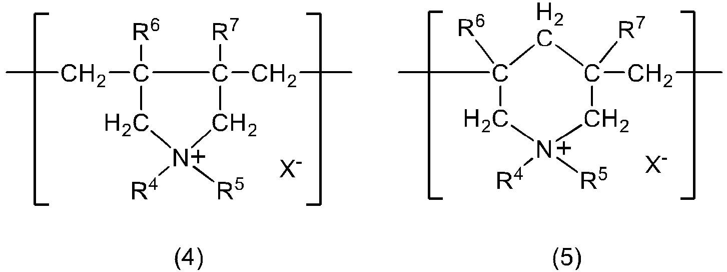 wherein, R⁴ and R⁵ may be the same or different and are a hydrogen atom, an alkyl group having 1 to 18 carbon atoms, an aryl group (such as a phenyl group), a hydroxyalkyl group, an amidoalkyl group, a cyanoalkyl group, an alkoxyalkyl group, or a carboalkoxyalkyl group, R⁶ and R⁷ may be the same or different and are a hydrogen atom, an alkyl group having 1 to 3 carbon atoms, or a phenyl group, and X⁻ is an anion (such as a chloride ion, a bromide ion, an iodide ion, a sulfate anion, a sulfonate anion, a methylsulfate anion, a phosphate anion, and a nitrate anion).

The polymer of the component (D) contains preferably from 85 to 100 mol%, more preferably from 90 to 100 mol%, and further more preferably from 95 to 100 mol% of a constituting unit of the formula (4) or (5) per molecule, from the viewpoint of enhancing the permeability to the deep of the head hair, the transferability into the inside of hair fibers, and the serum resistance to realize high dyeability without color unevenness.

The polymer of the component (D) is preferably a homopolymer of a diallyl quaternary ammonium salt or a copolymer of a diallyl quaternary ammonium salt and acrylic acid and is more preferably a copolymer of a diallyl quaternary ammonium salt and acrylic acid.

The copolymer of a diallyl quaternary ammonium salt and acrylic acid is preferably a copolymer of, for example, the following formula (4a) or (5a): 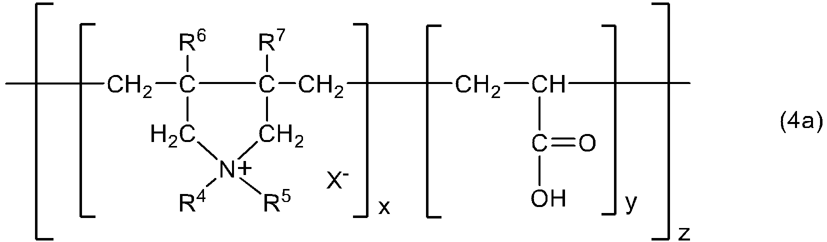 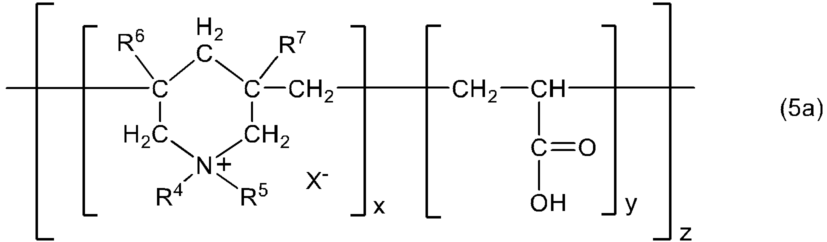 wherein, R⁴, R⁵, R⁶, R⁷, and X⁻ mean the same as above, x and y are each a number of from 1 to 99, and z is a number of from 150 to 8 000.

The ratio between x and y (x : y) is preferably from 85 : 15 to 99 : 1, more preferably from 90 : 10 to 99 : 1, and further more preferably from 95 : 5 to 99 : 1, from the viewpoint of enhancing the permeability to the deep of the head hair, the transferability into the inside of hair fibers, and the serum resistance to realize high dyeability without color unevenness.

The addition form of x and y may be block or random.

The component (D) preferably has a weight-average molecular weight of 10 000 or more, more preferably 50 000 or more, and further preferably 100 000 or more and preferably has 3 000 000 or less, further more preferably 1 000 000 or less, and further more preferably 200 000 or less, from the viewpoint of enhancing the permeability to the deep of the head hair, the transferability into the inside of hair fibers, and the serum resistance to realize high dyeability without color unevenness.

Here, the weight-average molecular weight can be measured by, for example, gel permeation chromatography (GPC) under the following conditions:
Mobile phase: 50 mM LiBr, 1 mass% CH₃COOH/ethanol : water = 3 : 7;
Column: TSK gel α-M (two columns connected in series); and
Reference material: polyethylene glycol.

Specific examples of the component (D) include MARCOAT 100 (manufactured by Lubrizol Advanced Materials, Inc., a homopolymer of diallyl quaternary ammonium) and MARCOAT series 280 and 295 (manufactured by Lubrizol Advanced Materials, Inc., a copolymer of a diallyl quaternary ammonium salt and acrylic acid).

The polymers as the component (D) can be used alone or in combination of two or more. The component (D) may be contained in any agent of the cosmetic product for bleaching or dyeing hair of the present invention but is preferably contained in the first part, from the viewpoint of enhancing the permeability to the deep of the head hair, the transferability into the inside of hair fibers, and the serum resistance to realize high dyeability without color unevenness.

The content of the component (D) in the liquid mixture is preferably 0.1 mass% or more, more preferably 0.2 mass% or more, and further more preferably 0.4 mass% or more and is preferably 2 mass% or less, more preferably 1 mass% or less, and further more preferably 0.5 mass% or less, from the viewpoint of enhancing the permeability to the deep of the head hair, the transferability into the inside of hair fibers, and the serum resistance to realize high dyeability without color unevenness.

### [Component (E): nonionic surfactant]

The cosmetic product for bleaching or dyeing hair of the present invention can contain a nonionic surfactant as a component (E) in the liquid mixture. Examples of the nonionic surfactant include polyoxyalkylene alkyl ether, alkyl alkanolamide, polyoxyalkylene alkylamine, alkyl polyglucoside, and alkyl glyceryl ether. In particular, from the viewpoint of the foaming and foam retention of the dispensed liquid mixture and the solubility of the dye contained in the cosmetic product for bleaching or dyeing hair, polyoxyalkylene alkyl ether is preferable.

As the polyoxyalkylene alkyl ether, those of the following formula (6) can be used,

R⁸-O-(AO)ᵣ-H (6)

wherein, R⁸ is a linear or branched, saturated or unsaturated hydrocarbon group having 8 to 22 carbon atoms, A is an alkylene group having 2 to 4 carbon atoms, and r is an average value and is a number of from 1 to 250.

The number of carbon atoms of R⁸ is preferably from 10 to 22, further preferably from 12 to 20, and further preferably from 12 to 18, from the viewpoint of the foaming and foam retention of the dispensed liquid mixture and the solubility of the dye contained in the cosmetic product for bleaching or dyeing hair.

A is preferably an ethylene group or a propylene group and more preferably an ethylene group.

r is preferably from 1 to 200, further preferably from 2 to 180, and further preferably from 10 to 160.

The polyoxyalkylene alkyl ether is preferably a combination of two or more compounds with different average numbers r of moles of alkylene oxide added, from the viewpoint of enhancing the permeability to the deep of the head hair, the transferability into the inside of hair fibers, and the serum resistance to realize high dyeability without color unevenness. That is, as the polyoxyalkylene alkyl ether, a combination of two compounds, a compound (E1) having r of 1 or more and 79 or less and a compound (E2) having r of 80 or more and 250 or less, is preferably used. In the component (E1), r is preferably 10 or more, more preferably 20 or more, and further more preferably 30 or more and is preferably 70 or less, more preferably 60 or less, and further more preferably 50 or less. In the component (E2), r is preferably 100 or more and more preferably 120 or more and is preferably 200 or less and more preferably 180 or less.

Specific examples of the polyoxyalkylene alkyl ether include polyoxyethylene lauryl ether, polyoxyethylene cetyl ether, polyoxyethylene stearyl ether, and polyoxyethylene oleyl ether. In particular, polyoxyethylene cetyl ether is preferable from the viewpoint of the foaming and foam retention of the dispensed liquid mixture and the solubility of the dye contained in the cosmetic product for bleaching or dyeing hair.

As the alkyl alkanolamide, those of the following formula (7) can be used, 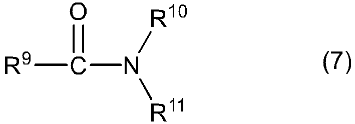 wherein, R⁹ is a linear or branched, saturated or unsaturated hydrocarbon group having 5 to 23 carbon atoms, and R¹⁰ and R¹¹ are each independently a hydrogen atom or an alkanol group having 1 to 4 carbon atoms, excluding that R¹⁰ and R¹¹ are simultaneously hydrogen atoms.

The number of carbon atoms of R⁹ is preferably from 8 to 22, further preferably from 10 to 20, and further preferably from 12 to 18, from the viewpoint of the foaming of the dispensed liquid mixture and the solubility of the dye contained in the cosmetic product for bleaching or dyeing hair.

Specific examples of the alkyl alkanolamide include coconut oil fatty acid monoethanolamide, coconut oil fatty acid diethanolamide, lauric acid isopropanolamide, and oleic acid diethanolamide.

As the polyoxyalkylene alkylamine, those of the following formula (8) can be used, 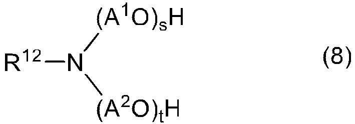 wherein, R¹² is a linear or branched, saturated or unsaturated hydrocarbon group having 5 to 23 carbon atoms, A¹ and A² are each independently an ethylene group or a propylene group, and s and t are each the average value, and the sum of both is from 5 to 50.

The number of carbon atoms of R¹² is preferably from 8 to 22, further preferably from 10 to 20, and further preferably from 12 to 18, from the viewpoint of the foaming of the dispensed liquid mixture and the solubility of the dye contained in the cosmetic product for bleaching or dyeing hair. A¹ and A² are preferably ethylene groups. The sum of s and t is preferably from 8 to 30 and more preferably from 10 to 20.

Specific examples of the polyoxyalkylene alkylamine include polyoxyethylene laurylamine, polyoxyethylene cetylamine, and polyoxyethylene cocamine.

The alkyl polyglucoside preferably includes an alkyl group having 6 to 22 carbon atoms and has a degree of glucoside unit condensation of from 1 to 7, and specific examples thereof include octyl polyglucoside, 2-ethylhexyl polyglucoside, decyl polyglucoside, lauryl polyglucoside, myristyl polyglucoside, palmityl polyglucoside, isostearyl polyglucoside, stearyl lauryl polyglucoside, oleyl polyglucoside, and behenyl polyglucoside. Among these polyglucosides, more preferred one includes an alkyl group having 8 to 18 carbon atoms and has a degree of glucoside unit condensation of from 1 to 7.

The alkyl glyceryl ether preferably includes an alkyl group having 8 to 20, further preferably 14 to 18, carbon atoms and preferably includes a branched alkyl group. Specific examples thereof include isostearyl glyceryl ether and isostearyl pentaerythryl glyceryl ether.

Among these nonionic surfactants, the polyoxyalkylene alkyl ether is preferable from the viewpoint of the stability of the cosmetic product for bleaching or dyeing hair and the solubility of the dye contained therein.

The nonionic surfactants as the component (E) can be used alone or in combination of two or more. The content of the component (E) in the liquid mixture is preferably 0.01 mass% or more, more preferably 0.1 mass% or more, and further more preferably 0.3 mass% or more and is preferably 3 mass% or less, more preferably 2 mass% or less, and further more preferably 1 mass% or less, from the viewpoint of enhancing the permeability to the deep of the head hair, the transferability into the inside of hair fibers, and the serum resistance to realize high bleachability or dyeability without color unevenness.

### [Dye]

When the cosmetic product for bleaching or dyeing hair of the present invention is a cosmetic product for hair dyeing, the first part contains an oxidation dye intermediate or a direct dye.

### (Oxidation dye intermediate)

As the oxidation dye intermediate, a known precursor and a coupler that are generally used in hair dye can be used. Examples of the precursor include paraphenylenediamine, toluene-2,5-diamine, orthochloroparaphenylenediamine, N-phenylparaphenylenediamine, N,N-bis(hydroxyethyl)paraphenylenediamine, 3-methyl-4-aminophenol, 2-hydroxyethyl paraphenylenediamine, paraaminophenol, paramethylaminophenol, 4-amino-metacresol, orthoaminophenol, 1-hydroxyethyl-4,5-diaminopyrazole, 2,4,5,6-tetraaminopyrimidine, 2,5,6-triamino-4-pyrimidinol, 2-methoxymethyl-paraphenylenediamine, and salts thereof.

Examples of the coupler include resorcin, 2-methylresorcin, 1-naphthol, 1,5-dihydroxynaphthalene, 5-aminoorthocresol, metaphenylenediamine, orthoaminophenol, metaaminophenol, paraaminophenol, 2,4-diaminophenoxyethanol, 2,6-diaminopyridine, 2-methyl-5-hydroxyethylaminophenol, 2-amino-3-hydroxypyridine, 4-chlororesorcin, and salts thereof.

Two or more kinds of each of the precursor and the coupler may be used in combination, and the contents of the precursor and the coupler in the first part are each preferably 0.005 mass% or more and more preferably 0.05 mass% or more and are each preferably 3 mass% or less and more preferably 2.5 mass% or less.

### (Direct dye)

Examples of the direct dye include an acid dye, a nitro dye, a disperse dye, and a basic dye. More specifically, examples of the acid dye include Blue No. 1, Violet No. 401, Black No. 401, Orange No. 205, Red No. 227, Red No. 106, Yellow No. 203, and Acid Orange No. 3; examples of the nitro dye include 2-nitro-p-phenylenediamine, 2-amino-6-chloro-4-nitrophenol, 3-nitro-p-hydroxyethylaminophenol, 4-nitro-o-phenylenediamine, 4-amino-3-nitrophenol, 4-hydroxypropylamino-3-nitrophenol, HC Blue 2, HC Orange 1, HC Red 1, HC Yellow 2, HC Yellow 4, HC Yellow 5, HC Red 3, and N,N-bis(2-hydroxyethyl)-2-nitro-p-phenylenediamine; examples of the disperse dye include Disperse Violet 1, Disperse Blue 1, and Disperse Black 9; and examples of the basic dye include Basic Blue 99, Basic Brown 16, Basic Brown 17, Basic Red 76, Basic Yellow 76, Basic Orange 31, and Basic Red 51. Furthermore, in addition to the above, picramic acid, Basic Violet 2, Basic Yellow 57, Basic Brown 16, Yellow No. 403, Basic Violet 2, Basic Yellow 87, HC Blue 18, HC Yellow 16, HC Red 16, Basic Blue 124, Yellow No. 4, HC Blue 15, and Tetrabromophenol Blue are also exemplified.

The direct dyes may be used in combination of two or more thereof or may be used in combination with an oxidation dye intermediate. The content of the direct dye in the first part is preferably 0.001 mass% or more and more preferably 0.01 mass% or more and is preferably 5 mass% or less and more preferably 3 mass% or less.

### [Oil agent]

The cosmetic product for bleaching or dyeing hair of the present invention can further contain an oil agent from the viewpoint of stabilizing the foam of the liquid mixture to be dispensed. Examples of the oil agent include hydrocarbons, such as squalene, squalane, liquid paraffin, liquid isoparaffin, and cycloparaffin; glycerides, such as castor oil, cocoa oil, mink oil, avocado oil, and olive oil; waxes, such as beeswax, spermaceti, lanolin, and carnauba wax; esters, such as isopropyl palmitate, isopropyl myristate, octyldodecyl myristate, hexyl laurate, cetyl lactate, propylene glycol monostearate, oleyl oleate, hexadecyl 2-ethylhexanoate, isononyl isononanoate, and tridecyl isononanoate; higher fatty acids, such as capric acid, lauric acid, myristic acid, palmitic acid, stearic acid, behenic acid, oleic acid, coconut oil fatty acid, isostearic acid, and isopalmitic acid; higher alcohols other than the components (A) and (B), such as cetyl alcohol, stearyl alcohol, and cetostearyl alcohol; and also isostearyl glyceryl ether and polyoxypropylene butyl ether.

The content of the oil agent in the liquid mixture is preferably 0.01 mass% or more, more preferably 0.03 mass% or more, and further more preferably 0.05 mass% or more and is preferably 3 mass% or less, more preferably 2.5 mass% or less, and further more preferably 2 mass% or less.

### [Silicones]

The cosmetic product for bleaching or dyeing hair of the present invention preferably does not contain silicones in the liquid mixture from the viewpoint of being capable of retaining the dispensed foam for a long period of time, but can further contain silicone in a certain range for smoothly applying the foam to the head head hair or for adding a high conditioning effect to the head hair. Examples of the silicone include dimethyl polysiloxane, methylphenyl polysiloxane, polyether-modified silicone, amino-modified silicone, oxazoline-modified silicone elastomer, and emulsions thereof dispersed in water by surfactants. Among them, polyether-modified silicone, amino-modified silicone, and emulsions thereof are preferable from the standpoint of being dispersible stably in water without use of a thickener.

The polyether-modified silicone includes end-modified silicone and side-chain-modified silicone, for example, pendant-type (comb-type), both end-modified-type, and one end-modified-type silicones. Examples of the modified silicone include a dimethylsiloxane-methyl(polyoxyethylene)siloxane copolymer, a dimethylsiloxane-methyl(polyoxypropylene)siloxane copolymer, and a dimethylsiloxane-methyl(polyoxyethylene-polyoxypropylene) siloxane copolymer. The polyether-modified silicone preferably has am HLB of 10 or more and further preferably an HLB of from 10 to 18 from the standpoint of compatibility with water. Here, HLB refers to a value determined from a cloud number (cloud number: an index correlated with HLB, applicable to an ether-type nonionic surfactant).

The amino-modified silicone may be any silicone having an amino group or an ammonium group, and is preferably amodimethicone.

In adding the silicones to the liquid mixture, the content of silicones is preferably 0.005 mass% or more and further more preferably 0.01 mass% or more from the viewpoint of smoothly applying the foam to the hair and adding a high conditioning effect to the hair, and is preferably 2 mass% or less, further preferably 1.5 mass% or less, and further preferably 1 mass% or less from the viewpoint of not preventing foaming of the dispensed liquid mixture.

### [Nonvolatile hydrophilic solvent]

Furthermore, the liquid mixture preferably contains a nonvolatile hydrophilic solvent. Consequently, it is possible to reduce the irritation to the scalp due to concentration of irritating components, such as hydrogen peroxide, through evaporation of the moisture from the liquid mixture after the application of the foam of the liquid mixture to the head hair and during leaving it to stand. As the nonvolatile hydrophilic solvent, for example, polyols and their lower (1 to 4 carbon atoms) alkyl ethers are preferable. The polyols preferably have 2 to 6 carbon atoms, and examples thereof include glycerol, diglycerol, propylene glycol, dipropylene glycol, 1,3-butanediol, ethylene glycol, diethylene glycol, isoprene glycol, and sorbitol. Examples of the lower alkyl ethers of the polyols include mono-lower alkyl ethers and poly-lower alkyl ethers of polyols (e.g., di-lower alkyl ether). In particular, a monomethyl ether or monoethyl ether of a polyol is preferable. Specifically, examples thereof include ethylene glycol monomethyl ether, ethylene glycol monoethyl ether, diethylene glycol monomethyl ether, and diethylene glycol monoethyl ether. These solvents may be used in combination of two or more.

From the standpoint of the effect of reducing the scalp irritation and the effect of improving the foam quality, the content of the nonvolatile hydrophilic solvent in the liquid mixture is preferably 0.1 mass% or more, more preferably 0.3 mass% or more, further more preferably 0.5 mass% or more, and further more preferably 1 mass% or more and is preferably 10 mass% or less, more preferably 5 mass% or less, further more preferably 3 mass% or less, and further more preferably 2 mass% or less.

### [Other optional component]

The cosmetic product for bleaching or dyeing hair of the present invention can contain another component that is used as a general cosmetic product raw material, in addition to the above components. Examples of these optional components include animal and vegetable oils and fats, natural and synthetic polymers, ethers, protein derivatives, hydrolyzed protein, amino acids, preservatives, chelating agents, stabilizers, antioxidants, plant extracts, herbal extracts, vitamins, fragrances, and UV absorbers.

### [pH]

The pH (25°C) of the liquid mixture of the cosmetic product for bleaching or dyeing hair of the present invention is preferably 8 or more and more preferably 9 or more and is preferably 12 or less, more preferably 11 or less, and further more preferably 10.5 or less, from the standpoint of a bleaching or dyeing effect and skin irritation. Examples of a pH adjuster include, in addition to the above alkali agents, an inorganic acid such as hydrochloric acid and phosphoric acid; an organic acid such as citric acid and, glycolic acid, and lactic acid; and a phosphoric acid salt such as monopotassium dihydrogen phosphate and disodium monohydrogen phosphate.

### [Viscosity]

The viscosity range of the liquid mixture is preferably 1 mPa·s or more and more preferably 5 mPa·s or more and is preferably 600 mPa·s or less, more preferably 300 mPa·s or less, further more preferably 200 mPa·s or less, and further more preferably 100 mPa·s or less. The viscosity herein is measured at 25°C by a B-type rotational viscometer (model: digital viscometer TV-10, Toki Sangyo Co., Ltd.) using rotor No. 1 at a rotation speed of 60 rpm for a measurement subject having a viscosity of 100 mPa·s or less, at a rotation speed of 30 rpm for a measurement subject having a viscosity of from 100 to 200 mPa·s, and at a rotation speed of 12 rpm for a measurement subject having a viscosity of 200 mPa·s or more. The measurements are performed in order of from the highest rotation speed to the lowest, and the measurement is completed at the point when the measurement is made without the display going off the scale, and measurements with smaller rotation speeds thereafter are not performed. The viscosity of the liquid mixture is measured 3 minutes after the start of mixing the respective agents.

A foam volume that can be easily applied can be realized by adjusting the viscosity of the liquid mixture in the above range, and dripping after the application of the liquid mixture to the head hair can be suppressed. In addition, squeezing to dispense foam with a squeeze foamer becomes easy, and pumping to dispense foam with a pump foamer becomes easy. In order to adjust the viscosity of the liquid mixture in the above range, a water-soluble solvent may be added, or the contents and the kinds of the surfactant, polyols, higher alcohols, and so on may be appropriately adjusted.

The cosmetic product for bleaching or dyeing hair of the present invention can improve the permeability to the deep of the hair while maintaining the viscosity low during from the dispense to the application. The viscosity of foam dispensed from a container is preferably 15 Pa·s or less, more preferably 13 Pa·s or less, and further more preferably 10 Pa·s or less during from the dispense to the application from the viewpoint of enhancing the permeability to the deep of the head hair, and is preferably 0.5 Pa·s or more, more preferably 1.0 Pa·s or more, more preferably 3.0 Pa·s or more, more preferably 4.0 Pa·s or more, and further more preferably 5.0 Pa·s or more from the viewpoint of preventing dripping.

Here, the viscosity of foam is measured by using a B-type viscometer (Helical, T-C, 10 rpm, 1 minute).

### [Container]

In the present invention, the container for dispensing the liquid mixture containing the first part and the second part as a foam is preferably a non-aerosol foaming container and is used for mixing the liquid mixture containing the first part and the second part with air without using a propellant and dispensing it as a foam. Use of a foaming container provides an effect of preventing scattering of the dispensed agent and also an effect of dispensing foam of the first part and the second part mixed uniformly. In particular, since a product of a non-aerosol type container can be manufactured inexpensively compared to that of an aerosol type container, and since there is no need for a highpressure gas propellant, the product can be handled more safely during distribution.

As the foaming container, a known pump foaming container, squeeze foaming container, electric whisk, accumulator pump foaming container, or the like that has a foam dispensing means can be used. More specifically, examples of the foaming container include pump foamer E3 type and F2 type (Daiwa Can Company) described in Food & Packaging (vol. 35, No. 10, pp. 588 to 593 (1994); vol. 35, No. 11, pp. 624 to 627 (1994); vol. 36, No. 3, pp. 154 to 158 (1995)), a squeeze foamer (Daiwa Can Company), an electric whisk (Panasonic Corporation), and an air spray foamer (Air Spray International). The foaming container that is used in the cosmetic product for bleaching or dyeing hair of the present invention is preferably a pump foaming container or a squeeze foaming container because of their inexpensiveness and good usability.

The pump foaming container or the squeeze foaming container includes a foam-generating portion such as a net and preferably includes a thin net because when the liquid mixture of the first part and the second part is dried and solidified and causes clogging, the solidified material is immediately dissolved by the flow of foam by the dispense at the next time and the clogging is cleared. In this case, the mesh of the net is preferably from 50 to 350 mesh, further preferably from 70 to 280 mesh, further preferably from 90 to 250 mesh, and further preferably from 130 to 220 mesh. Here, the mesh refers to the number of openings per inch. The use of a net with mesh in this range allows generation of creamy foam. As the material of such a mesh, nylon, polyester, and so on can be preferably exemplified.

The foaming container that is used in the cosmetic product for bleaching or dyeing hair of the present invention is arranged with at least one net, preferably a plurality of nets, and is particularly preferably arranged with two nets from the standpoint of economy, foam stability, and so on.

In the foaming container, the portions coming into contact with the contents (the inner wall of the container, the inner wall of the foam dispense means, and so on) are preferably made of a material that is not corroded by an alkali and hydrogen peroxide and allows the oxygen generated by decomposition of hydrogen peroxide to permeate therethrough.

The product of the cosmetic product for bleaching or dyeing hair of the present invention including a first part, a second part, and a foaming container may be in a form in which the first part and the second part are filled in respective containers other than the foaming container, and both parts are put and mixed in the foaming container upon application or may be a form in which one of the parts is filled in the foaming container, the other part is filled in another container, and the other part is put in the foaming container upon application. In this case, the second part is preferably filled in a container with gas permeability, in particular, a foaming container that is made of a material (e.g., polyethylene) with oxygen permeability in order to prevent the pressure in the container from increasing due to oxygen generated by decomposition of hydrogen peroxide. In contrast, for the first part, it is required to use a container that hardly allows oxygen to permeate in order to prevent oxidation of the oxidation dye.

The mix ratio of the first part and the second part in the cosmetic product for bleaching or dyeing hair of the present invention is preferably from 1 : 4 to 4 : 1 in the mass ratio and further preferably from 1 : 3 to 1 : 1.

### [Gas-liquid mix ratio]

The gas-liquid mix ratio of air and the liquid mixture by the foam dispensing means of the foaming container is preferably from 5 to 40 mL/g and more preferably from 6 to 30 mL/g from the standpoint of ease of familiarity and ease of application of the agent to the head hair. The gas-liquid mix ratio herein is the value measured as follows.

The gas-liquid mix ratio is determined by measuring the mass and volume of foam dispensed at 25°C. One hundred grams of a liquid mixture is placed into a squeeze foaming container (Daiwa Can Company, capacity: 210 mL, mesh roughness (opening): 150 mesh (150 squares per inch (25.4 mm), in the mixing chamber) and 200 mesh (in the top)), 20 g of foam is dispensed into a 1 000-mL measuring cylinder when the remaining amount becomes 80 g, and the foam volume is measured 1 minute after the start of dispensing. The gas-liquid mix ratio (mL/g) is obtained by dividing the volume (mL) of the dispensed foam by the mass, i.e., 20 g.

### [Use method]

In order to bleach or dye the hair with the cosmetic product for bleaching or dyeing hair of the present invention, the hair is preferably combed in advance. Consequently, since the hair is unlikely to tangle during re-foaming as described later, there is no risk of scattering of the liquid mixture. In addition, blocking, which is widely performed in the application of compositions for bleaching or dyeing, is not required to be performed after combing the hair, and furthermore blocking is preferably not performed. Consequently, the operation of applying a composition for bleaching or dyeing to the hair and the operation of re-foaming described later become easier. In addition, a finish without color unevenness is obtained further more by the operation of re-foaming even when the cosmetic product for bleaching or dyeing hair of the present invention is applied to hair with a hairdressing. Subsequently, the first part and the second part of the cosmetic product for bleaching or dyeing hair of the present invention are mixed in the foaming container. The agent dispensed as a foam from the container may be directly applied to hair or may be applied to hair with a hand or with a tool such as a brush. From the viewpoint of preventing scattering or dripping of an agent, it is preferable to take the agent in a (gloved) hand and apply it to hair.

After the application, the liquid mixture is left to stand for from about 3 to about 60 minutes, preferably for from about 5 to 45 minutes. On this occasion, from the viewpoint of further preventing dripping during being left to stand and sufficiently distributing the liquid mixture also to the roots of the hair, it is preferable to perform re-foaming on the head hair. Re-foaming may be performed by injecting gas, by using a tool such as a vibrator or a brush, or by using fingers. More preferably, it is performed by finger rubbing.

Here, re-foaming may be performed after the foam completely disappears, while the foam disappears, or before the applied foam changes. Alternatively, re-foaming may be performed after completion of application of the foam to the entire range of the area to which foam is intended to be applied or during the application. Re-foaming may be performed continuously once or intermittently repeated multiple times.

In addition, rubbing with fingers for re-foaming can serve to increase the viscosity of the foam notably, and it is possible to suppress the hair from flying off and to prevent the foam from dripping. The viscosity of the foam after rubbing is preferably 15 Pa·s or more and more preferably 22 Pa·s or more from the viewpoint of suppressing the hair from flying off and preventing dripping.

After these operations, the liquid mixture is rinsed off. Subsequently, the hair is appropriately shampooed and conditioned, and then rinsed with water, and the hair is dried.

Regarding the above embodiments, preferable aspects of the present invention will be further disclosed below.
<1> A cosmetic product for bleaching or dyeing hair, comprising a first part containing an alkali agent, a second part containing hydrogen peroxide, and a container for dispensing a liquid mixture including the first part and the second part as a foam, wherein
at least one of all agents, including the first part and the second part, before mixing is a solubilizing solution,
the liquid mixture contains components (A) to (D) below, and
assuming (A) contained in the solubilizing solution before mixing as component (A)s, a mass ratio of the component (A)s to the sum of the component (A) and the component (B), (A)s/[(A) + (B)], is 0.079 or less,
   (A) an aliphatic alcohol having 10 or more and 14 or less carbon atoms;
   (B) an aliphatic alcohol having 19 or more and 24 or less carbon atoms;
   (C) an ionic surfactant; and
   (D) polymer including a diallyl quaternary ammonium salt as a constituting unit.
<2> The cosmetic product for bleaching or dyeing hair according to <1>, wherein the alkali agent is preferably one or more selected from the group consisting of ammonia and a salt thereof, an alkanolamine and a salt thereof, an alkanediamine and a salt thereof, and a carbonate.
<3> The cosmetic product for bleaching or dyeing hair according to <1> or <2>, wherein the content of the alkali agent in the liquid mixture is preferably 1.0 mass% or more, more preferably 1.5 mass% or more, and further more preferably 2.0 mass% or more and is preferably 20 mass% or less, more preferably 15 mass% or less, further more preferably 10 mass% or less, and further more preferably 5 mass% or less.
<4> The cosmetic product for bleaching or dyeing hair according to any one of <1> to <3>, wherein the content of hydrogen peroxide in the liquid mixture is preferably 0.5 mass% or more, more preferably 1 mass% or more, and further more preferably 2 mass% or more and is preferably 6 mass% or less and more preferably 5 mass% or less.
<5> The cosmetic product for bleaching or dyeing hair according to any one of <1> to <4>, wherein the component (A) is preferably one or more selected from the group consisting of decanol, dodecanol, and myristyl alcohol and is more preferably myristy alcohol.
<6> The cosmetic product for bleaching or dyeing hair according to any one of <1> to <5>, wherein the content of the component (A) in the liquid mixture is preferably 0.1 mass% or more, more preferably 0.15 mass% or more, and further more preferably 0.2 mass% or more and is preferably 2 mass% or less, more preferably 1 mass% or less, and further more preferably 0.5 mass% or less.
<7> The cosmetic product for bleaching or dyeing hair according to any one of <1> to <6>, wherein the component (B) preferably includes one or more selected from the group consisting of arachyl alcohol, behenyl alcohol, and carnaubyl alcohol and more preferably includes behenyl alcohol.
<8> The cosmetic product for bleaching or dyeing hair according to any one of <1> to <7>, wherein the content of the component (B) in the liquid mixture is preferably 0.05 mass% or more, more preferably 0.1 mass% or more, and further more preferably 0.2 mass% or more and is preferably 2 mass% or less, more preferably 1.5 mass% or less, and further more preferably 1 mass% or less.
<9> The cosmetic product for bleaching or dyeing hair according to any one of <1> to <8>, wherein the total content of the aliphatic alcohols in the solubilizing solution is preferably 0.3 mass% or less, more preferably 0.2 mass% or less, further more preferably 0.1 mass% or less, further more preferably 0.05 mass% or less, further more preferably 0.01 mass% or less, further more preferably 0.005 mass% or less, and further more preferably 0.001 mass% or less, or the solubilizing solution does not include an aliphatic alcohol.
<10> The cosmetic product for bleaching or dyeing hair according to any one of <1> to <9>, assuming the component (A) contained in the solubilizing solution before mixing as component (A)s, the mass ratio of the component (A)s to the sum of the component (A) and the component (B), (A)s/[(A) + (B)], is preferably 0.07 or less, more preferably 0.06 or less, further more preferably 0.05 or less, further more preferably 0.04 or less, further more preferably 0.03 or less, further more preferably 0.02 or less, further more preferably 0.01 or less, further more preferably 0.005 or less, further more preferably 0.001 or less, further more preferably 0.0005 or less, and further more preferably 0.0001 or less, and alternatively the solubilizing solution does not contain the component (A).
<11> The cosmetic product for bleaching or dyeing hair according to any one of <1> to <10>, wherein a component (C) is one or more selected from the group consisting of a cationic surfactant, an anionic surfactant, and an amphoteric surfactant.
<12> The cosmetic product for bleaching or dyeing hair according to <11>, wherein
   the cationic surfactant is a combination of a long-chain monoalkylammonium salt of the following formula (2) : 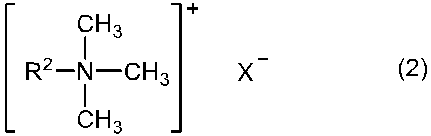
   wherein, R² is a linear alkyl group having 14 to 16 carbon atoms, and X⁻ is an anion (such as a chloride ion, a bromide ion, an iodide ion, a sulfate anion, a sulfonate anion, a methylsulfate anion, a phosphate anion, and a nitrate anion), and
   a long-chain monoalkylammonium salt of the following formula (3): 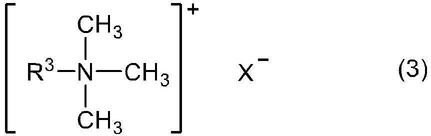 wherein, R³ is a linear alkyl group having 18 to 24 carbon atoms, and X⁻ means the same as above.
<13> The cosmetic product for bleaching or dyeing hair according to <11> or <12>, wherein the anionic surfactant is one or more selected from the group consisting of a sulfate anionic surfactant, a carboxylic acid anionic surfactant, a sulfonic acid anionic surfactant, and a phosphate anionic surfactant and is more preferably one or more selected from the group consisting of a carboxylic acid anionic surfactant and a sulfate anionic surfactant.
<14> The cosmetic product for bleaching or dyeing hair according to any one of <11> to <13>, wherein the amphoteric surfactant is preferably one or more selected from the group consisting of carbobetaine, amidobetaine, sulfobetaine, hydroxysulfobetaine, amidosulfobetaine, phosphobetaine, and imidazolinium surfactants having an alkyl group, alkenyl group, or acyl group having 8 to 24 carbon atoms, more preferably one or more selected from the group consisting of a carbobetaine surfactant and a sulfobetaine surfactant, and further more preferably one or more selected from the group consisting of lauramidopropyl betaine, coconut oil fatty acid amidopropyl betaine, lauryldimethylaminoacetic acid betaine, and laurylhydroxysulfobetaine.
<15> The cosmetic product for bleaching or dyeing hair according to any one of <1> to <14>, wherein the content of the component (C) in the liquid mixture is preferably 0.5 mass% or more, more preferably 1 mass% or more, further more preferably 1.5 mass% or more, and further more preferably 2 mass% or more and is preferably 7 mass% or less, more preferably 5 mass% or less, further more preferably 4 mass% or less, and further more preferably 3 mass% or less.
<16> The cosmetic product for bleaching or dyeing hair according to any one of <1> to <15>, wherein the charge density of the component (D) is preferably 4.0 meq/g or more, more preferably 4.3 meq/g or more, further more preferably 4.6 meq/g or more, further more preferably 4.9 meq/g or more, further more preferably 5.2 meq/g or more, and further more preferably 5.5 meq/g or more and is preferably 8.0 meq/g or less, more preferably 7.0 meq/g or less, and further more preferably 6.5 meq/g or less.
<17> The cosmetic product for bleaching or dyeing hair according to any one of <1> to <16>, wherein the component (D) is one or more selected from the group consisting of a homopolymer of a diallyl quaternary ammonium salt and a copolymer of a diallyl quaternary ammonium salt and acrylic acid.
<18> The cosmetic product for bleaching or dyeing hair according to any one of <1> to <17>, wherein the content of the component (D) in the liquid mixture is preferably 0.1 mass% or more, more preferably 0.2 mass% or more, and further more preferably 0.4 mass% or more and is preferably 2 mass% or less, more preferably 1 mass% or less, and further more preferably 0.5 mass% or less.
<19> The cosmetic product for bleaching or dyeing hair according to any one of <1> to <18>, wherein the liquid mixture further contains, as a component (E), one or more selected from the group consisting of a polyoxyalkylene alkyl ether, an alkyl alkanolamide, a polyoxyalkylene alkylamine, an alkyl polyglucoside, and an alkyl glyceryl ether, more preferably a polyoxyalkylene alkyl ether nonionic surfactant.
<20> The cosmetic product for bleaching or dyeing hair according to <19>, wherein the polyoxyalkylene alkyl ether is an ether of the following formula (6):

   R⁸-O-(AO)ᵣ-H (6)

   wherein, R⁸ is a linear or branched, saturated or unsaturated hydrocarbon group having 12 to 18 carbon atoms, A is an ethylene group, and r is an average value and is a number of from 10 to 160.
<21> The cosmetic product for bleaching or dyeing hair according to <20>, wherein the polyoxyalkylene alkyl ether includes a component (E1) having r of 1 or more and 79 or less and a component (E2) having r of 80 or more and 250 or less, wherein the r of the component (E1) is 30 or more and 50 or less, and the r of the component (E2) is 120 or more and 180 or less.
<22> The cosmetic product for bleaching or dyeing hair according to any one of <19> to <21>, wherein the content of the component (E) in the liquid mixture is preferably 0.01 mass% or more, more preferably 0.1 mass% or more, and further more preferably 0.3 mass% or more and is preferably 3 mass% or less, more preferably 2 mass% or less, and further more preferably 1 mass% or less.
<23> The cosmetic product for bleaching or dyeing hair according to any one of <1> to <22>, wherein the liquid mixture further contains an oil agent selected from the group consisting of hydrocarbons, glycerides, waxes, esters, higher fatty acids, isostearyl glyceryl ether, higher alcohols other than the components (A) and (B), and polyoxypropylene butyl ether.
<24> The cosmetic product for bleaching or dyeing hair according to <23>, wherein the content of the oil agent in the liquid mixture is preferably 0.01 mass% or more, more preferably 0.03 mass% or more, and further more preferably 0.05 mass% or more and is preferably 3 mass% or less, more preferably 2.5 mass% or less, and further more preferably 2 mass% or less.
<25> The cosmetic product for bleaching or dyeing hair according to any one of <1> to <24>, wherein the liquid mixture further contains, as a nonvolatile hydrophilic solvent, one or more selected from the group consisting of polyols having 2 to 6 carbon atoms and lower (1 to 4 carbon atoms) alkyl ethers thereof.
<26> The cosmetic product for bleaching or dyeing hair according to <25>, wherein the content of the nonvolatile hydrophilic solvent in the liquid mixture is preferably 0.1 mass% or more, more preferably 0.3 mass% or more, further more preferably 0.5 mass% or more, and further more preferably 1 mass% or more and is preferably 10 mass% or less, more preferably 5 mass% or less, further more preferably 3 mass% or less, and further more preferably 2 mass% or less.
<27> The cosmetic product for bleaching or dyeing hair according to any one of <1> to <26>, wherein the viscosity of the foam dispensed from the container during from the dispensing to the application is preferably 15 Pa·s or less, more preferably 13 Pa·s or less, and further more preferably 10 Pa·s or less and is preferably 0.5 Pa·s or more, more preferably 1.0 Pa·s or more, more preferably 3.0 Pa·s or more, more preferably 4.0 Pa·s or more, and further more preferably 5.0 Pa·s or more.
<28> The cosmetic product for bleaching or dyeing hair according to any one of <1> to <27>, wherein the container is a non-aerosol foaming container.
<29> A hair bleaching or hair dyeing method comprising using the cosmetic product for bleaching or dyeing hair according to any one of <1> to <28> to dispense a liquid mixture containing the first part and the second part as a foam from the container, and applying the foam to hair by a hand.
<30> The hair bleaching or hair dyeing method according to <29>, further comprising performing rubbing with fingers after application of the foam dispensed from the container to the hair, wherein the viscosity of the foam after rubbing is preferably 22 Pa·s or more and more preferably 22.5 Pa·s or more.

### EXAMPLES

### Examples 1 to 8 and Comparative Example 1

The first part (solubilizing solution) and the second part (emulsion) of each formulation shown in Table 2 were prepared and were mixed in a mass ratio of 1 : 1.5 to give respective liquid mixtures. The liquid mixtures were each dispensed as a foam with a squeeze foamer (Daiwa Can Company, capacity: 210 mL, mesh roughness: 150 mesh (in the mixing chamber) and 200 mesh (in the top), total opening area of the narrowest part of the air inlet: 0.35 mm², inner diameter ϕ of dip tube: 1.75 mm), and the following evaluations were performed.

### 1. Evaluation of foam permeability

### (Evaluation method)

An expert panelist discharged one push of foam (approximately 3 g) in the hand from the squeeze foaming container and smoothed the foam onto medium length hair of a mannequin by about 20 cm from the top of the head towards the ends of the hair three times while applying a force of about 600 g. This operation was repeated twice. The inner side at a position about 5 cm from the top of the head was subjected to slicing (operation of parting the hair and opening the inner side) with the pointed grip of a typical ring comb (tail comb), and the permeation state of the foam was verified visually and evaluated by the following criteria. The results are shown in Table 2.

### (Evaluation criteria)

A: the foam is applied uniformly to the deep;
B: although it is not uniform, the foam is partially applied to about half of the area;
C: although it is not uniform, the foam is partially applied in places;
D: a small amount of the foam permeates, but almost no foam is applied; and
E: no foam permeates at all.

### 2. Evaluation of sebum resistance

### (Evaluation method)

To the entire scalp of a mannequin having a medium hair length was applied about 0.8 g of glyceryl tricaprylate as a pseudo sebum and 0.6 g of "Liese Playful Care Oil" manufactured by Kao Corporation as a general hair oil.

An expert panelist dispensed foam from the above squeeze foaming container, applied 80 g of the foam (a liquid mixture of 32 g of the first part and 48 g of the second part) in total to a mannequin while parting the hair, and then rubbed the foam for several seconds. Subsequently, the hair was left to stand for 20 minutes at room temperature and subjected to slicing, and the foam remaining state was verified and was evaluated by the following criteria. The results are shown in Table 2.

### (Evaluation criteria)

A: foam remains uniformly in the deep;
B: foam partially disappears and remains nonuniformly;
C: foam almost disappears and does not remain; and
D: foam does not remain at all, and liquid drips.

### 3. Evaluation of foam viscosity

### (Evaluation method)

Approximately 10 g of foam was dispensed from the above squeeze foaming container to a wig with a hair length of about 1 to 2 cm and was applied all over the wig to spread the foam to the hair. Subsequently, the foam was rubbed on the wig until the increase in the foam viscosity is saturated.

The foam (a) dispensed, the foam (b) after thorough application, and the foam (c) after sufficiently rubbed were transferred to respective beakers, and the foam viscosities were measured by using a B-type viscometer (Helical, T-C, 10 rpm, 1 minute).

### (Evaluation criteria)

The foam was verified to be "OK" or "NG" from the foam viscosity values at the above three stages in accordance with the following criteria, and was verified from A to D in accordance with the aspects shown in Table 1. The results are shown in Table 2.
(a): a viscosity of 10 Pa·s or less is judged as OK, and a viscosity of higher than 10 Pa·s is judged as NG;
(b): a viscosity of 15 Pa·s or less is judged as OK, and a viscosity of higher than 15 Pa·s is judged as NG; and
(c): a viscosity of 22 Pa·s or more is judged as OK, and a viscosity of less than 22 Pa·s is judged as NG.

**[Table 1]**

| | A | B | B~C | C | C | D | D |
|---|---|---|---|---|---|---|---|
| (a) | OK | OK | NG | OK | NG | NG | NG |
| (b) | OK | OK | OK | NG | OK | NG | NG |
| (c) | OK | NG | OK | OK | NG | OK | NG |

**[Table 2]**

| | First part (mass%) | Example 1 | Example 2 | Example 3 | Example 4 | Example 5 | Example 6 | Example 7 | Example 8 | Comparative Example 1 |
|---|---|---|---|---|---|---|---|---|---|---|
| Component A | Myristyl alcohol (*1) | - | 0.016 | 0.065 | 0.080 | 0.095 | 0.120 | 0.200 | 0.300 | 0.140 |
| Component C | Sodium cocoyl glutamate liquid (1)(25%)(*2) | 8 | 8 | 8 | 8 | 8 | 8 | 8 | 8 | 8 |
| Component C | Sodium polyoxyethylene lauryl ether sulfate (2.0E.O.)(27%)(*3) | 12 | 12 | 12 | 12 | 12 | 12 | 12 | 12 | 12 |
| Component D | Diallyldimethylammonium chloride-acrylic acid copolymer liquid (37%)(*4) | 3 | 3 | 3 | 3 | 3 | 3 | 3 | 3 | 3 |
| Dye | Paraaminophenol | 0.203 | 0.203 | 0.203 | 0.203 | 0.203 | 0.203 | 0.203 | 0.203 | 0.203 |
| | Metaaminophenol | 0.494 | 0.494 | 0.494 | 0.494 | 0.494 | 0.494 | 0.494 | 0.494 | 0.494 |
| | Toluene-2,5-diamine liquid (20%) | 9.865 | 9.865 | 9.865 | 9.865 | 9.865 | 9.865 | 9.865 | 9.865 | 9.865 |
| | Resorcin | 1.287 | 1.287 | 1.287 | 1.287 | 1.287 | 1.287 | 1.287 | 1.287 | 1.287 |
| | 2,4-Diaminophenoxyethanol Hydrochloride | 0.072 | 0.072 | 0.072 | 0.072 | 0.072 | 0.072 | 0.072 | 0.072 | 0.072 |
| | 5-Aminoorthocresol | 0.145 | 0.145 | 0.145 | 0.145 | 0.145 | 0.145 | 0.145 | 0.145 | 0.145 |
| Alkali agent | Monoethanolamine | 3.5 | 3.5 | 3.5 | 3.5 | 3.5 | 3.5 | 3.5 | 3.5 | 3.5 |
| | Ammonium chloride | 1.7 | 1.7 | 1.7 | 1.7 | 1.7 | 1.7 | 1.7 | 1.7 | 1.7 |
| | Strono ammonia water (28%) | 0.6 | 0.6 | 0.6 | 0.6 | 0.6 | 0.6 | 0.6 | 0.6 | 0.6 |
| Nonvolatile hydrophilic solvent | Propylene alycol | 2.5 | 2.5 | 2.5 | 2.5 | 2.5 | 2.5 | 2.5 | 2.5 | 2.5 |
| | Polypropylene glycol (*5) | 2.5 | 2.5 | 2.5 | 2.5 | 2.5 | 2.5 | 2.5 | 2.5 | 2.5 |
| Others | Ascorbic acid | 0.4 | 0.4 | 0.4 | 0.4 | 0.4 | 0.4 | 0.4 | 0.4 | 0.4 |
| | Exsiccated sodium sulfate | 0.105 | 0.105 | 0.105 | 0.105 | 0.105 | 0.105 | 0.105 | 0.105 | 0.105 |
| | Tetrasodium edetate dihydrate | 0.1 | 0.1 | 0.1 | 0.1 | 0.1 | 0.1 | 0.1 | 0.1 | 0.1 |
| | Ethanol | 5 | 5 | 5 | 5 | 5 | 5 | 5 | 5 | 5 |
| | Fraarance | 0.75 | 0.75 | 0.75 | 0.75 | 0.75 | 0.75 | 0.75 | 0.75 | 0.75 |
| | Sodium chloride | 4 | 4 | 4 | 4 | 4 | 4 | 4 | 4 | 4 |
| | Purified water | Balance | Balance | Balance | Balance | Balance | Balance | Balance | Balance | Balance |
| Total | | 100.000 | 100.000 | 100.000 | 100.000 | 100.000 | 100.000 | 100.000 | 100.000 | 100.000 |

| | Second part (mass%) | | | | | | | | | |
|---|---|---|---|---|---|---|---|---|---|---|
| Component A | Myristyl alcohol (*1) | 0.41 | 0.41 | 0.39 | 0.39 | 0.39 | 0.38 | 1.05 | 1.58 | 0.37 |
| Component B | Behenyl alcohol (*6) | 0.63 | 0.62 | 0.60 | 0.60 | 0.59 | 0.58 | 1.62 | 2.42 | 0.57 |
| Component C | Cetyltrimethylammonium chloride (30%)(*7) | 0.66 | 0.66 | 0.66 | 0.66 | 0.66 | 0.66 | 0.66 | 0.66 | 0.66 |
| Component C | Stearyltrimethylammonium chloride (28%)(*8) | 1.42 | 1.42 | 1.42 | 1.42 | 1.42 | 1.42 | 1.42 | 1.42 | 1.42 |
| Component E | Polyoxyethylene (150) cetyl ether (*9) | 0.6 | 0.6 | 0.6 | 0.6 | 0.6 | 0.6 | 0.6 | 0.6 | 0.6 |
| Component E | Polyoxyethylene (40) cetyl ether (*10) | 0.36 | 0.36 | 0.36 | 0.36 | 0.36 | 0.36 | 0.36 | 0.36 | 0.36 |
| Hydrogen peroxide | Hydrogen peroxide solution (35%) | 16.29 | 16.29 | 16.29 | 16.29 | 16.29 | 16.29 | 16.29 | 16.29 | 16.29 |
| Oil agent | Soft lanoline fatty acid (*11) | 0.06 | 0.06 | 0.06 | 0.06 | 0.06 | 0.06 | 0.06 | 0.06 | 0.06 |
| Nonvolatile hydrophilic solvent | Propylene glycol | 0.6 | 0.6 | 0.6 | 0.6 | 0.6 | 0.6 | 0.6 | 0.6 | 0.6 |
| | Polypropylene glycol (*5) | 0.75 | 0.75 | 0.75 | 0.75 | 0.75 | 0.75 | 0.75 | 0.75 | 0.75 |
| Others | Hydroxyethanediphosphonic acid liquid (60%)(*12) | 0.067 | 0.067 | 0.067 | 0.067 | 0.067 | 0.067 | 0.067 | 0.067 | 0.067 |
| | Sodium hydroxide liquid | 0.04 | 0.04 | 0.04 | 0.04 | 0.04 | 0.04 | 0.04 | 0.04 | 004 |
| | Purified water | Balance | Balance | Balance | Balance | Balance | Balance | Balance | Balance | Balance |
| Total | | 100.000 | 100.000 | 100.000 | 100.000 | 100.000 | 100.000 | 100.000 | 100.000 | 100.000 |
| Solubilizing solution | Component (A)s | 0.00 | 0.02 | 0.07 | 0.08 | 0.10 | 0.12 | 0.20 | 0.30 | 0.14 |
| Liquid mixture | Component A | 0.25 | 0.25 | 0.26 | 0.27 | 0.27 | 0.28 | 0.71 | 1.07 | 0.28 |
| | Component B | 0.38 | 0.37 | 0.36 | 0.36 | 0.35 | 0.35 | 0.97 | 1.45 | 0.34 |
| | Component C | 2.45 | 2.45 | 2.45 | 2.45 | 2.45 | 2.45 | 2.45 | 2.45 | 2.45 |
| | (A)s/[(A)+(B)] | 0.000 | 0.010 | 0.042 | 0.051 | 0.061 | 0.077 | 0.048 | 0.048 | 0.090 |
| Evaluation of permeability of foam | | A | A | A | B | B | B | A | A | C |
| Evaluation of sebum resistance | | A | A | A | A | A | A | B | C | A |
| Evaluation of foam viscosity | | A | B | B | B | B | C | B | B | D |

| | | | | | | | | | | |
|---|---|---|---|---|---|---|---|---|---|---|
| [0149] *1: Kalcol 4098 (manufactured by Kao Corporation) ***2:** Amisoft CS22B (manufactured by Kao Corporation) ***3:** Emal 227 pH 11 (manufactured by Kao Corporation) ***4:** MARCOAT 295 (manufactured by Lubrizol Advanced Materials) *5: Adeka Carpol DL-30 (manufactured by Adeka Corporation) *6: Kalcol 220-80 (manufactured by Kao Corporation) *7: QUARTAMIN 60W (manufactured by Kao Corporation) *8: QUARTAMIN 86W (manufactured by Kao Corporation) ***9:** Nikkol BC-150 (manufactured by Nikko Chemicals **Co., Ltd.)** *10: Nikkol BC-40TX (manufactured by Nikko Chemicals **Co., Ltd.)** *11: 18MEA-SO-(RB) (manufactured by Croda) ***12:** Dequest 2010CS (manufactured by Italmatch Japan **Ltd.)** | | | | | | | | | | |

## Claims

1. A cosmetic product for bleaching or dyeing hair, comprising a first part containing an alkali agent, a second part containing hydrogen peroxide, and a container for dispensing a liquid mixture including the first part and the second part as a foam, wherein
at least one of all agents, including the first part and the second part, before mixing is a solubilizing solution,
the liquid mixture contains components (A) to (D) below, and
assuming the component (A) contained in the solubilizing solution before mixing as component (A)s, a mass ratio of the component (A)s to the sum of the component (A) and the component (B), (A)s/[(A) + (B)], is 0.079 or less,
(A) an aliphatic alcohol having 10 or more and 14 or less carbon atoms;
(B) an aliphatic alcohol having 19 or more and 24 or less carbon atoms;
(C) an ionic surfactant; and
(D) a polymer including a diallyl quaternary ammonium salt as a constituting unit.

2. The cosmetic product for bleaching or dyeing hair according to Claim 1, wherein a content of the aliphatic alcohol in the solubilizing solution is 0.3 mass% or less.

3. The cosmetic product for bleaching or dyeing hair according to Claim 1 or 2, wherein the container is a non-aerosol foaming container.

4. A hair bleaching or hair dyeing method comprising using the cosmetic product for bleaching or dyeing hair according to any one of Claims 1 to 3 to dispense a liquid mixture containing the first part and the second part as a foam from the container, and applying the foam to hair with a hand.
